# EUROPÄISCHE PATENTSCHRIFT

(11) **EP 3 272 331 B1**
(45) Veröffentlichungstag und Bekanntmachung des Hinweises auf die Patenterteilung: **04.07.2018**
(21) Anmeldenummer: 16180762.3
(22) Anmeldetag: 22.07.2016
(51) Int. Cl.: A61K 8/89, C08G 77/38

(54) **VERFAHREN ZUR HERSTELLUNG VON SILOXANEN ENTHALTEND GLYCERINSUBSTITUENTEN**
METHOD FOR THE PREPARATION OF SILOXANES-CONTAINING GLYCERIN SUBSTITUENTS
PROCEDE DE PRODUCTION DE SILOXANES CONTENANT DES SUBSTITUTS DE GLYCERINE

(43) Veröffentlichungstag der Anmeldung: 24.01.2018
(73) Patentinhaber: Evonik Degussa GmbH, 45128 Essen (DE)
(72) Erfinder: KNOTT, Wilfried, 45355 Essen (DE); DUDZIK, Horst, 45326 Essen (DE); KLEIN, Klaus-Dieter, 45481 Mülheim an der Ruhr (DE)

(56) Entgegenhaltungen:
- EP-A1- 2 243 799
- US-A1- 2016 052 849

## Beschreibung

### Gebiet der Erfindung

Gegenstand der Erfindung ist ein Verfahren zur Herstellung von Siloxanen, welche Glycerin-Modifikationen sowie gleichzeitig hydrophobe Substituenten aufweisen. Ein weiterer Gegenstand der vorliegenden Erfindung ist ein Siloxan, welches Glycerin-Modifikationen und auch hydrophobe Seitenketten aufweist, wobei die Glycerin-Modifikationen mindestens teilweise Ketalgruppen tragen, sowie die Verwendung dieser neuen Siloxane.

### Stand der Technik

Organomodifizierte Siloxane werden in den verschiedensten Applikationen eingesetzt. Ihre Eigenschaften lassen sich durch die Art der Modifikation sowie durch die Modifikationsdichte gezielt einstellen.

Die Edelmetall-katalysierte Hydrosilylierungsreaktion stellt eine universelle Methodik zur SiC-verknüpfenden Modifizierung SiH-Gruppen aufweisender Siloxangerüste dar.

So können auf diese Weise zum Beispiel mit Allylpolyethern organophile oder nichtionische hydrophile Gruppen an ein Siloxangerüst gebunden werden. Derartige Verbindungen finden ihren Einsatz zum Beispiel als Polyurethan-Schaum-Stabilisatoren und als Entschäumer in Treibstoffen. Durch Umsetzung mit α-Olefinen kann dagegen das Siloxan mit lipophilen Gruppen verknüpft werden. Die somit erhältlichen Siliconwachse dienen zum Beispiel als Additive in kosmetischen Anwendungen.

Da polyetherhaltige Verbindungen in letzter Zeit zunehmend in Kritik geraten sind, besteht Bedarf an siloxanbasierten Emulgatoren, die keine Polyethergruppen tragen, gleichzeitig aber über gute Emulgier- und Dispergiereigenschaften verfügen.

Hierfür wurden Glycerin- oder Polyglycerin-Derivate genutzt, um als hydrophile Komponente die Polyethergruppen zu ersetzen. Solche polyglycerinmodifizierten Siloxane werden beispielsweise in EP 1213316 beschrieben. In der Anmeldung EP 1550687 werden glycerinmodifizierte Siloxane für die Anwendung in Emulsionen eingesetzt.

Die EP 1550687 offenbart, dass man glycerinmodifizierte, insbesondere polyglycerinmodifizierte Siloxane direkt durch Hexachloroplatinsäure-katalysierte SiC-Verknüpfung seitständiger Wasserstoffsiloxane mit Polyglycerinmono- respektive Polyglycerindiallylether in Isopropanol als Reaktionsmedium herstellen kann. Hierbei entstehen zwangsläufig SiOC-verknüpfte, hydrolyselabile Nebenprodukte.

Die EP2243799 widmet sich dieser Problematik und lehrt, dass man die Schwächen bis dato bekannter Systeme dadurch vermeiden kann, indem man die Anknüpfung des Glycerinderivats nicht kammständig, sondern an den Termini des zu modifizierenden Siloxangerüstes vornimmt. Dennoch stellt die in dieser Schrift vorgestellte Lehre keine technische Lösung für die Herstellung hochreiner glycerinmodifizierter Siloxane dar. Der dort beschriebenen allgemeinen Arbeitsvorschrift zur Herstellung von Alkyl/Glycerin-modifizierten Siloxanen folgend, werden stöchiometrische Überschüsse an α-Olefin und Glycerinmonoallylether sowie 10 ppm Karstedt-Katalysator in ca. 30% Toluol (bezogen auf die Gesamteinwaage) unter Rühren vorgelegt und auf 95 °C erhitzt. Das Wasserstoffsiloxan wird dann innerhalb von 2 Stunden bei 95°C zugetropft. Nach destillativer Aufarbeitung des vollständigen SiH-Umsatz aufweisenden Reaktionsansatzes (Abziehen flüchtiger Anteile bei 110°C und einem angelegten Hilfsvakuuum von ca. 1 mbar) wird ein viskoses, leicht trübes, fast farbloses Produkt erhalten.

Die Verwendung des aromatischen Lösungsmittels gereicht diesem Verfahren zum Nachteil, wenn das erhaltene Siloxan als kosmetisches Additiv eingesetzt werden soll. Zusätzlich entstehen durch die Reaktionsführung, bei der ein ambidentes Substrat wie der Glycerinmonoallylether mit dem SiH-Siloxan in Gegenwart eines Platinkatalysators zusammengebracht wird, hydrolytisch instabile SiOC-verknüpfte Nebenprodukte. Diese Nebenprodukte sind aufgrund ihrer chemischen Verwandtschaft zu den Hauptprodukten und ihrer Nichtflüchtigkeit nicht vom Produkt abtrennbar und verbleiben somit darin. Immerhin macht bis zu ca. 25 Val-% des eingesetzten Glycerinmonoallylethers Nebenproduktbildung aus.

Als Folge bleibt die erzielbare Selektivität der SiC-Verknüpfung immer weit hinter der theoretisch erwarteten zurück.

Die Schwierigkeit, die aus der Präsenz des ambidenten Substrates resultiert, erkennend, stellt die Lehre der WO 2011134869 auf die Verwendung von 1,2-O-Isopropyliden-3-allyloxy-1,2-propandiol als Modifizierungsreagenz kammartig strukturierter SiH-Siloxane ab (Beispiele 1a, 2a, 3a). Obwohl nur der Ketalkörper an das jeweilige Wasserstoffsiloxan als einziger Substituent anzulagern ist, ist der Einsatz einer großen Menge an Dioxan notwendig (ca. 78% in Beispiel 1a, ca. 71% in Beispiel 2a und ca. 57% in Beispiel 3a), um nach mehr als 24 Stunden Ausbeuten von 88 bis 92% des noch zu entschützenden glycerinmodifizierten Siloxans zu isolieren.

Der Aufgabe gewidmet, diacrylatgruppentragende Siloxane für den Einsatz in strahlenhärtenden Systemen bereitzustellen, geht die US 4.908.228 auf die Reaktion von 1,2-O-Isopropyliden-3-allyloxy-1,2-propandiol mit Wasserstoffsilanen und -siloxanen ein und beschränkt sich dabei auf die Betrachtung rein binärer Stoffsysteme. Die erhaltenen glycerinketalmodifizierten Silane bzw. Siloxane werden anschließend einer Hydrolyse bzw. Alkoholyse unterworfen und die freigesetzten diolmodifizierten Siloxane werden acryliert. US2016/0052849 beschreibt die Herstellung eines Glycerinmodifizierten Siloxans, wobei ein deketalisiertes Allylglycerinderivat mit einem Organowasserstoffsiloxan umgesetztz wird. Aufgabe der Erfindung war es, ein Verfahren bereitzustellen, mit welchem sich ohne Einsatz von größeren Mengen an Lösungsmittel glycerinmodifizierte Siloxane herstellen lassen, welche zusätzlich hydrophobe Substituenten aufweisen.

### Beschreibung der Erfindung

Überraschenderweise wurde gefunden, dass das im Folgenden beschriebene Verfahren zur Herstellung von Siloxanen, welche Glycerin-Modifikationen sowie gleichzeitig hydrophobe Substituenten aufweisen, ohne große Lösungsmittelmengen durchgeführt werden kann. Völlig überraschend ergibt sich bei den im erfindungsgemäßen Verfahren eingesetzten Edukten eine unvorhersehbar hohe Phasenkompatibilität. Üblicherweise sind lösemittelfrei durchgeführte Hydrosilylierungsreaktionen von einer substratabhängigen Inkompatibilität geprägt, die dazu führt, dass die SiC-Verknüpfungsreaktion aus der Mehrphasigkeit, im Regelfall aus einer Zweiphasigkeit, startet. Die SiH-Gruppen aufweisende Silikonphase und die anzulagernden olefinischen Reaktionspartner sind durch ihre unterschiedlichen Polaritäten zueinander nicht affin und zudem noch durch Dichteunterschiede separiert. So ist beispielhaft die lösemittelfreie SiC-Verknüpfungsreaktion zwischen SiH-Siloxanen und ungesättigten (Poly)ethern anzuführen, die stets zweiphasig beginnt. Mit der einsetzenden Bildung des SiC-verknüpften Copolymers entsteht dann ein phasenkompatibilisierendes Tensid, das an der Phasengrenze zur Dispergierung der Reaktionspartner beiträgt. Mit der im Reaktionsverlauf steigenden Produktkonzentration sinkt die Konzentration inkompatibler Edukte, die in Form kleiner Tröpfchen die Reaktionsmatrix durchsetzen. Am sogenannten Klarpunkt hat der Durchmesser der individuellen Tröpfchen der inkompatiblen dispergierten Phase die Wellenlänge sichtbaren Lichts unterschritten und die zuvor trübe Reaktionsmatrix erscheint dem unbewaffneten Auge als eine klare Phase.

Die praktischen Erfahrungen bei der SiC-verknüpfenden (Poly)ether-Modifizierung von Siloxanen kennend, stellt die bei der Herstellung der erfindungsgemäß beanspruchten, gleichzeitig Glycerin- , Glycerinketal und hydrophobe Substituenten tragenden Siloxane beobachtete Phasenkompatibilität der Addenden im Hydrosilylierungsschritt eine Überraschung für den Fachmann dar, denn formal kann man Glycerinmonoallyletherketal - losgelöst von seiner Herstellung und von Nomenklaturfragen - als eine ungesättigte (Poly)etherverbindung betrachten, die 3 Ethersauerstoffatome aufweist. An die Stelle der sonst bei (Poly)etheranlagerungen beobachteten Zweiphasigkeit der Reaktionsmatrix tritt - und das von Beginn der Hydrosilylierung an - eine klare, mit bloßem Auge nicht zu differenzierende einheitliche Phase. Für die betriebliche Praxis ist dieses Systemverhalten von außerordentlicher Bedeutung, da es die Notwendigkeit vermeidet, Lösemittel einzusetzen und/ oder die Reaktionspartner aufwendig sequenziert an das Polymergerüst anzulagern, wie es zum Beispiel im Production Example 1 der WO2013103147, Seite 49 ff. beschrieben wird.

Gegenstand der vorliegenden Erfindung ist daher ein Verfahren zur Herstellung von modifizierten Siloxanen umfassend die Verfahrensschritte
A) Bereitstellen eines Wasserstoffsiloxans aufweisend pro Molekül mindestens drei SiH-Gruppen;
B) Hydrosilylierung des Wasserstoffsiloxans mit
   mindestens einem α-olefinischen Kohlenwasserstoff aufweisend 2 bis 36, insbesondere 8 bis 28, Kohlenstoffatome und
   mindestens einem ketalisierten Glycerinderivat, welches umfasst:
   ein Glyceringerüst und
   eine α-olefinischen Kopplungsgruppe; und gegebenenfalls
C) Aufreinigung des hydrosilylierten Siloxans; und gegebenenfalls
D) mindestens teilweise saure Hydrolyse der in den mit dem Wasserstoffsiloxan reagierten ketalisierten Glycerinderivaten enthaltenen Ketale.

Ein weiterer Gegenstand der Erfindung ist ein modifiziertes Siloxan erhältlich nach dem vorgenannten Verfahren, sowie die Verwendung desselben als Emulgator.

Ein Vorteil des vorliegenden, erfindungsgemäßen Verfahrens ist es, dass sich die glycerinmodifizierten Siloxane in Blick auf SiOC-verknüpfte Nebenprodukte hochrein darstellen lassen.
Ein weiterer Vorteil des erfindungsgemäßen Verfahrens ist, es dass die erhältlichen glycerinmodifizierten Siloxane hervorragend Emulsionen und Dispersionen zu stabilisieren vermögen. Solche Formulierungen sind lagerstabil, und die Emulsionen oder Dispersionen zeigen über eine Zeit von mehreren Monaten und bei unterschiedlichen Temperaturen eine konstant stabile Zusammensetzung und Homogenität ohne Öl- oder Wasser- oder Feststoff-Separation. Insbesondere vorteilhaft erweisen sich die die erhältlichen glycerinmodifizierten Siloxane speziell bei kosmetischen Emulsionspräparaten für Make-Up und Sonnenschutzanwendungen, die zusätzlich anorganische Pigmente wie zum Beispiel Titandioxid oder Eisenoxide enthalten weil die erhältlichen glycerinmodifizierten Siloxane diesen Formulierungen eine gute Langzeitstabilität und eine sehr gute Pigmentdispergierung verleihen.
Ein weiterer Vorteil des erfindungsgemäßen Verfahrens ist, es dass es eine konstante und reproduzierbare Qualität des Endprodukts liefert, welche nach den bisher bekannten technischen Verfahren zur Herstellung glycerinmodifizierter Siloxane nur schwer, wenn überhaupt, zu erreichen war.

Das erfindungsgemäße Herstellverfahren sowie die erfindungsgemäßen glycerinmodifizierten Siloxane werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt vollständig zum Offenbarungsgehalt der vorliegenden Erfindung gehören. Werden im Rahmen der vorliegenden Erfindung Verbindungen, wie z. B. Siloxane, beschrieben, die verschiedene Einheiten mehrfach aufweisen können, so können diese statistisch verteilt (statistisches Oligomer) oder geordnet (Blockoligomer) in diesen Verbindungen vorkommen. Angaben zu Anzahl von Einheiten in solchen Verbindungen sind als Mittelwert, gemittelt über alle entsprechenden zu verstehen, da in der Regel insbesondere Siloxane als Mischungen vorliegen.
Alle angegebenen Prozent (%) sind, wenn nicht anders angegeben, Massenprozent.

Das erfindungsgemäße Verfahren zur Herstellung von modifizierten Siloxanen ist insbesondere gerichtet auf die Herstellung von Siloxanen, welche mindestens eine längerkettige Kohlenwasserstoff-Gruppe sowie mindestens eine Glycerin aufweisende Gruppe aufweisen. Unter dem Begriff "Wasserstoffsiloxan" oder "H-Siloxan" im Zusammenhang mit der vorliegenden Erfindung ist immer SiH-funktionelles Siloxan zu verstehen. Die erfindungsgemäß eingesetzten Wasserstoffsiloxane sind durch die dem Fachmann bekannten Verfahren, etwa der Equilibrierung, wie zum Beispiel in US7196153 beschrieben, erhältlich.
Unter dem Begriff "ketalisierte" Glycerinderivate im Zusammenhang mit der vorliegenden Erfindung ist zu verstehen, dass zum Glycerinanteil gehörige OH-Gruppen des Glycerinderivates formal durch Reaktion mit mindesten einem Keton in ein Ketal überführt worden sind. Bei den ketalisierten Glycerinderivaten ist in diesem Zusammenhang mindestens ein Teil der OH-Gruppen ketalisiert; bevorzugt sind mindestens 5%, insbesondere mindestens 15% der OH-Gruppen ketalisiert.

Die Struktur des Siloxangrundgerüstes des mit dem erfindungsgemäßen Verfahren herzustellenden modifizierten Siloxans wird offensichtlich durch das in Verfahrensschritt A) eingesetzte Wasserstoffsiloxan bestimmt.
Das modifizierte Siloxan weist bevorzugt 20 bis 300, besonders bevorzugt 50 bis 150 Si-Atome pro Molekül auf.
Es können im Siloxangerüst lineare, verzweigte und auch dendritische Siloxane mit dem erfindungsgemäßen Verfahren modifiziert werden.
Entsprechende dendritische Siloxane, welche durch partielle Hydrosilylierung von Wasserstoffsiloxanen mit Olefingruppen tragenden, verzweigenden Siloxanen wie z.B Vinyltris(trimethylsiloxy)silan gewonnen und in Verfahrensschritt B) eingesetzt werden können, sind beispielsweise in der WO2013103147beschrieben.
So kann man in Analogie zur Lehre der WO2013103147ein monoglycerinmodifiziertes dendritisches Siloxan gemäß vorliegender Erfindung gewinnen, indem man Vinyltris(trimethylsiloxy)silan, ein α-Olefin und Glycerinketalmonoallylether hydrosilylierend beispielsweise an ein seitständiges Wasserstoffsiloxan anlagert und das Anlagerungsprodukt danach einer partiellen Deketalisierung unterwirft.

Die Anlagerung der olefinischen Reaktionspartner im Verfahrensschritt B) kann in beliebiger Reihenfolge sequenziert oder aber bevorzugt konzertiert erfolgen.

Ein bevorzugtes erfindungsgemäßes Verfahren dient zur Herstellung von modifizierten Siloxanen der allgemeinen Formel I)

M_{2+h+2i-a-b-c} M'ₐ M"_{b} M'"_{c} D_{d} D'ₑ D"_{f} D'"_{g} Tₕ Qᵢ allgemeine Formel I)

mit
- M: = (R¹₃SiO_{1/2}),
- M': = (R¹₂R²SiO_{1/2}),

- M": = (R¹₂R³ Si O_{1/2}),
- M'": = (R¹₂R⁴ Si O_{1/2}),
- D: = (R¹₂ Si O_{2/2}),
- D': = (R¹R² Si O_{2/2}),
- D": = (R¹R³ Si O_{2/2}),
- D'": = (R¹R⁴ Si O_{2/2}),
- T: = (R¹SiO_{3/2}) und
- Q: = (Si O_{4/2}),
wobei
R¹ = unabhängig voneinander gleiche oder verschiedene lineare oder verzweigte, gegebenenfalls aromatische Kohlenwasserstoffreste mit 1 bis 16, bevorzugt 1 bis 7, Kohlenstoffatomen, die gegebenenfalls OH- oder Esterfunktionen tragen, oder Tris(trimethylsiloxy)silanethyl, bevorzugt Methyl, Phenyl oder Tris(trimethylsiloxy)silanethyl, insbesondere Methyl oder Tris(trimethylsiloxy)silanethyl,
R² = unabhängig voneinander gleiche oder verschiedene lineare oder verzweigte, gegebenenfalls aromatische Kohlenwasserstoffreste mit 2 bis 36, bevorzugt 8 bis 28, Kohlenstoffatomen, bevorzugt lineare Alkylreste mit 8 bis 28 Kohlenstoffatomen, insbesondere n-Dodecyl oder n-Hexadecyl, und
R³ = unabhängig voneinander gleich oder verschieden, ausgewählt aus glycerinhaltiger Rest, R⁴ = unabhängig voneinander gleich oder verschieden, ausgewählt aus ketalisierter glycerinhaltiger Rest,
ist,
mit
- a: = 0 bis 7 , insbesondere 0 bis 3,
- b: = 0 bis 7, insbesondere 0 bis 3,
- c: = 0 bis 7, insbesondere 0 bis 3,
- d: = 10 bis 200, bevorzugt 20 bis 150, insbesondere 50 bis 130,
- e: = 1 bis 130, bevorzugt 5 bis 80, insbesondere 8 bis 40,
- f: = 0 bis 75, bevorzugt 1 bis 45, insbesondere 1 bis 25,
- g: = 0 bis 75, bevorzugt 1 bis 45, insbesondere 4 bis 25,
- h: = 0 bis 10, bevorzugt 0 bis 5, insbesondere 0
- i: = 0 bis 5, bevorzugt 0 bis 2, insbesondere 0,
mit der Maßgabe, dass c + g > 0.

Daraus ergibt sich für das erfindungsgemäße Verfahren, wie oben bereits erläutert, dass in Verfahrensschritt A) bevorzugt solche Wasserstoffsiloxan bereitgestellt werden, die der oben genannten allgemeinen Formel I) entsprechen, bei denen R², R³ und R⁴ = H bedeuten.

Es ist offenbar, dass der in Verfahrensschritt B) eingesetzte α-olefinische Kohlenwasserstoff die Struktur des Restes R² und das in Verfahrensschritt B) eingesetzte ketalisierte Glycerinderivat die Struktur des Restes R³ und R⁴ bestimmt.
Somit werden beispielsweise in Verfahrensschritt B) als α-olefinische Kohlenwasserstoffe bevorzugt lineare α-Alkene mit 8 bis 28 Kohlenstoffatomen, insbesondere n-Dodecene oder n-Hexadecene eingesetzt.
Es ist offenbar, dass im Zusammenhang mit der vorliegenden Erfindung der Rest R³ ein glycerinhaltiger Rest ist, der - klar abgrenzbar zu R⁴ - nicht ketalisiert ist.

Unter dem Begriff "glycerinhaltiger Rest" im Zusammenhang mit der vorliegenden Erfindung ist ein Substituent zu verstehen, der die Struktur -OCH₂CH(O-)CH₂O- umfasst.
Analoge Strukturen sind unter dem Begriff "Glyceringerüst" im Zusammenhang mit der vorliegenden Erfindung zu verstehen.

In Verfahrensschritt B) kann die Hydrosilylierung des Wasserstoffsiloxans mit dem α-olefinischen Kohlenwasserstoff und dem ketalisierten Glycerinderivat gleichzeitig oder getrennt voneinander, d.h. in beliebiger Reihenfolge hintereinander, durchgeführt werden, wobei es erfindungsgemäß bevorzugt ist, wenn in Verfahrensschritt B) die Hydrosilylierung des Wasserstoffsiloxans mit einer Mischung enthaltend den α-olefinischen Kohlenwasserstoff und das ketalisierte Glycerinderivat durchgeführt wird.

In Verfahrensschritt B) kann die Hydrosilylierung nach etablierten Methoden in Gegenwart eines Katalysators durchgeführt werden. Dabei können beispielsweise Katalysatoren eingesetzt werden wie Platin-, Rhodium-, Osmium-, Ruthenium-, Palladium-, Iridium-Komplexe oder ähnliche Verbindungen bzw. die entsprechenden reinen Elemente oder deren auf Silica, Aluminiumoxid oder Aktivkohle oder ähnlichen Trägermaterialien immobilisierten Derivate. Bevorzugt wird die Hydrosilylierung mit Hilfe von Platinkomplexen wie cis-(NH₃)₂PtCl₂-(Cis-Platin), Di-µ-[chloro-bis chloro(cyclohexen)platin(II)] oder [Tris(divinyltetramethyldisiloxan)bis-platin(0)] (Karstedt-Katalysator) und besonders bevorzugt mit Olefin-aktivierten Platin(0)-Komplexkatalysatoren (sog. WK-Katalysatoren) gemäß der Lehre der EP1520870 durchgeführt. Die Katalysatormenge wird hierbei so bemessen, dass die Gesamtkonzentration an Platin von 1 bis 100 wppm bezogen auf das gesamte Reaktionsgemisch beträgt. Für den Fachmann ersichtlich, wird die minimale Platinkonzentration so gewählt, dass sie eine sicher-zügige SiC-Verknüpfungsreaktion gestattet, ohne die Wirtschaftlichkeit des Verfahrens durch zu hohen Edelmetalleinsatz zu beinträchtigen oder darüber hinaus auch noch nachteilige Produktverfärbungen hervorzurufen. Die Hydrosilylierung kann bei Temperaturen zwischen 0 und 200 °C, bevorzugt zwischen 50 und 140 °C, durchgeführt werden. Die Reaktion kann in geeigneten Lösungsmitteln wie aliphatischen oder aromatischen Kohlenwasserstoffen, cyclischen Oligosiloxanen, Alkoholen, Carbonaten oder Estern durchgeführt werden. Geeignete Verfahren zur Hydrosilylierung werden zum Beispiel im Buch "Chemie und Technologie der Silicone", Verlag Chemie, 1960, Seite 43, sowie in US3775452 und EP1520870 beschrieben, auf welche ausdrücklich verwiesen wird.
Im Gegensatz zu den bisher bekannten Verfahren kann in Verfahrensschritt B) auf den Einsatz eines Lösungsmittels verzichtet werden. Somit ist es erfindungsgemäß bevorzugt, dass in Verfahrensschritt B) maximal 20 Gew.-%, bevorzugt maximal 10 Gew.-%, insbesondere maximal 5 Gew.-%, Lösungsmittel eingesetzt wird, wobei sich die Gewichtsprozente auf die Summe von Wasserstoffsiloxan, α-olefinischem Kohlenwasserstoff, ketalisiertem Glycerinderivat sowie Lösungsmittel beziehen.

Das in Verfahrensschritt B) bevorzugt eingesetzte ketalisierte Glycerinderivat leitet sich bevorzugt strukturell durch Ketalisierung mit Substanzen ausgewählt aus der Gruppe R'-(CO)-R" mit R' und R" gleiche oder verschiedene Kohlenwasserstoffreste, insbesondere Alkylgruppen, mit 1 bis 10 Kohlenstoffatomen, Benzaldehyd, Cyclopentanon, Cyclohexanon und Cycloheptanon,
ab.
Die Siedepunktsdifferenzen der eingesetzten Ketalisierungsreagenzien und von deren Folgeprodukten berücksichtigend, hat es sich in der Praxis als vorteilhaft erwiesen, wenn man die Herstellung des ketalisierten Glycerinderivates mit dem entsprechenden Keton selber, sondern mit dessen entsprechenden Ketalen vollzieht. Hierbei sind bevorzugt Dimethoxy- oder Diethoxy-Ketale einzusetzen.
Verfahren zur Herstellung von zyklischen Acetalen und Ketalen werden in der DE19648960 und in der DE19647395 offenbart. Anleitungen zur Durchführung der Ketalisierung an Feststoffkatalysatoren werden beispielsweise in der DE102008015756 offenbart. Die WO2009010527 stellt auf ein Verfahren zur Herstellung von zyklischen Glycerinacetalen oder zyklischen Glycerinketalen oder Gemischen derselben ab.

In Verfahrensschritt B) wird bevorzugt ein ketalisiertes Glycerinderivat eingesetzt, welches die allgemeine Formel II) aufweist: 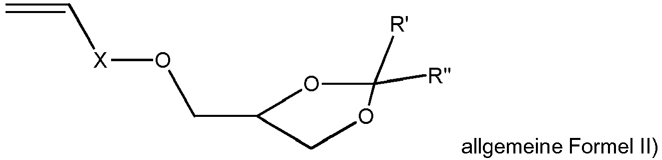 mit R' und R" gleiche oder verschiedene Kohlenwasserstoffreste, insbesondere Alkylgruppen, mit 1 bis 20 insbesondere 1 bis 4, Kohlenstoffatomen, wobei R' und R" kovalent über eine C-C-Bindung miteinander verbunden sein können, und
X ausgewählt aus zweibindigen Kohlenwasserstoffresten, insbesondere Alkylengruppen, mit 1 bis 10 Kohlenstoffatomen, wobei X = CH₂ besonders bevorzugt ist.

In dem Fall, dass R' und R" kovalent über eine C-C-Bindung miteinander verbunden sind, beträgt die Summe der Kohlenstoffatome von R' und R" bevorzugt 4 bis 7 C-Atome, ganz besonders bevorzugt 4 C-Atome, insbesondere bevorzugt liegt R' und R" zusammen in Form einer Alkylengruppe mit 4 bis 7 C-Atomen vor.
Ganz besonders bevorzugt wird in Verfahrensschritt B) ein ketalisiertes Glycerinderivat der allgemeinen Formel II) mit X = CH₂ und R' und R" = CH₃ eingesetzt.
Insbesondere bevorzugt wird vorgenanntes, bevorzugtes ketalisiertes Glycerinderivat in Verbindung mit einem erfindungsgemäßen Verfahren zur Herstellung von Siloxanen der allgemeinen Formel I) mit
- R¹: = Methyl,
- R²: = insbesondere n-Dodecyl oder n-Hexadecyl,
und
- a: = 0 bis 3,
- b: = 0 bis 3,
- c: = 0 bis 3,
- d: = 50 bis 130,
- e: = 8 bis 40,
- f: = 1 bis 25,
- g: = 0,5 bis 25, bevorzugt 1 bis 25,
- h: = 0, und
- i: = 0.
eingesetzt.

Es kann von Vorteil sein, wenn nach dem Verfahrensschritt B) das modifizierte Siloxan aufgereinigt wird. Dies wird bevorzugt durch Abdestillation von überschüssigen α-olefinischen Kohlenwasserstoffen und ketalisierten Glycerinderivaten und gegebenenfalls Lösungsmittel erreicht.

In Verfahrensschritt C) werden die in dem modifizierten Siloxan enthaltenen Ketale gegebenenfalls mindestens teilweise sauer hydrolysiert. Entsprechende Anleitungen zur Durchführung der sauren Hydrolyse werden beispielsweise in der WO2011134869 offenbart.
Die Hydrolyse erfolgt bevorzugt durch Zugabe einer wässrigen, sauren Lösung, insbesondere einer Toluolsulfonsäurelösung. Es ist vorteilhaft und daher erfindungsgemäß bevorzugt, wenn Verfahrensschritt C) bei einer Temperatur von 40 °C bis 100 °C durchgeführt wird. Erfindungsgemäß aller möglichen bevorzugt wird Verfahrensschritt C) derart ausgeführt, dass der Ketalisierungsgrad zwischen 5 % und 70%, bevorzugt 10 % und 40 %, besonders bevorzugt 15 % und 25 % liegt, wobei sich die Prozente auf alle im Siloxan vorhandenen, zur Ketalisierung geeigneten Diole beziehen. Der Ketalisierungsgrad lässt sich mit Hilfe der ¹H-NMR-Spektroskopie bestimmen.

Erfindungsgemäß bevorzugt kann das erfindungsgemäße Verfahren einen weiteren Verfahrensschritt D) umfassen, in dem das modifizierte Siloxan aufgereinigt wird. Verfahrensschritt D) umfasst erfindungsgemäß bevorzugt eine Neutralisation der in Verfahrensschritt C) zur sauren Hydrolyse eingesetzten Säure, gefolgt von einer Abtrennung des somit gebildeten Salzes. Dies erfolgt erfindungsgemäß bevorzugt in Form einer Abfiltration des Salzes. Zusätzlich ist erfindungsgemäß bevorzugt eine destillative Abtrennung der weiteren in Verfahrensschritt C) hinzugefügten Substanzen und gebildeten Nebenprodukte vorgesehen. Diese umfassen insbesondere Wasser und Ketone.

Ein weiterer Gegenstand der vorliegenden Erfindung sind Siloxane erhältlich nach dem erfindungsgemäßen Verfahren, die dadurch gekennzeichnet sind, dass sie mindestens einen ketalisierten glycerinhaltigen Rest aufweisen.

Insbesondere erfindungsgemäß bevorzugt werden modifizierte Siloxane der allgemeinen Formel I)

M_{2+h+2i-a-b-c} M'a M"_{b} M'"c D_{d} D'e D"_{f} D'"_{g} Tₕ Qᵢ allgemeine Formel I)

mit
- M: = (R¹₃ Si O_{1/2}),
- M': = (R¹₂ R² Si O_{1/2}),
- M": = (R¹2R³ Si O_{1/2}),
- M'": = (R¹₂R⁴ Si O_{1/2}),
- D: = (R¹₂ Si O_{2/2}),
- D': = (R¹R² Si O_{2/2}),
- D": = (R¹R³Si O_{2/2}),
- D'": = (R¹R⁴ Si O_{2/2}),
- T: = (R¹ Si O_{3/2}) und
- Q: = (Si O_{4/2}),
wobei
R¹ = unabhängig voneinander gleiche oder verschiedene lineare oder verzweigte, gegebenenfalls aromatische Kohlenwasserstoffreste mit 1 bis 16, bevorzugt 1 bis 7, Kohlenstoffatomen, die gegebenenfalls OH- oder Esterfunktionen tragen, oder Tris(trimethylsiloxy)silanethyl, bevorzugt Methyl, Phenyl oder Tris(trimethylsiloxy)silanethyl, insbesondere Methyl oder Tris(trimethylsiloxy)silanethyl,
R² = unabhängig voneinander gleiche oder verschiedene lineare oder verzweigte, gegebenenfalls aromatische Kohlenwasserstoffreste mit 2 bis 36, bevorzugt 8 bis 28, Kohlenstoffatomen, bevorzugt lineare Alkylreste mit 8 bis 28 Kohlenstoffatomen, insbesondere n-Dodecyl oder n-Hexadecyl, und
R³ = unabhängig voneinander gleich oder verschieden, ausgewählt aus glycerinhaltiger Rest,
R⁴ = unabhängig voneinander gleich oder verschieden, ausgewählt aus ketalisierter glycerinhaltiger Rest,
ist,
mit
- a: = 0 bis 7 , insbesondere 0 bis 3,
- b: = 0 bis 7, insbesondere 0 bis 3,
- c: = 0 bis 7, insbesondere 0 bis 3,
- d: = 10 bis 200, bevorzugt 20 bis 150, insbesondere 50 bis 130,
- e: = 1 bis 130, bevorzugt 5 bis 80, insbesondere 8 bis 40,
- f: = 0 bis 75, bevorzugt 1 bis 45, insbesondere 1 bis 25,
- g: = 0 bis 75, bevorzugt 1 bis 45, insbesondere 4 bis 25,
- h: = 0 bis 10, bevorzugt 0 bis 5, insbesondere 0
- i: = 0 bis 5, bevorzugt 0 bis 2, insbesondere 0,
mit der Maßgabe, dass c + g > 0.

Insbesondere bevorzugte erfindungsgemäße Siloxane sind dadurch gekennzeichnet, dass in der allgemeinen Formel I)
R¹ = Methyl,
R² = insbesondere n-Dodecyl oder n-Hexadecyl, und
R³ = 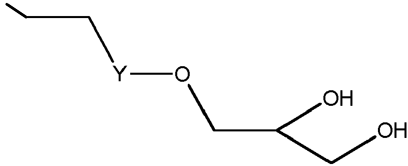 mit Y = [CH₂]ₛ, wobei s = 1 bis 5, insbesondere 1,
R⁴ = 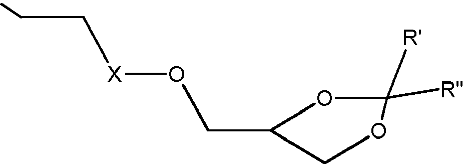 mit X = [CH₂]ᵣ, wobei r = 1 bis 5, insbesondere 1, und R' und R" = unabhängig voneinander gleich oder verschieden, linear Alkyl mit 1 bis 4 Kohlenstoffatomen, wobei R' und R" kovalent über eine C-C-Bindung miteinander verbunden sein können, insbesondere CH₃,
und
- a: = 0 bis 3,
- b: = 0 bis 3,
- c: = 0 bis 3,
- d: = 50 bis 130,
- e: = 8 bis 40,
- f: = 1 bis 25,
- g: = 0,5 bis 25, bevorzugt 1 bis 25,
- h: = 0, und
- i: = 0.
ist.

Die erfindungsgemäßen modifizierten Siloxane lassen sich vorteilhaft als Emulgator verwenden, da sie hervorragende oberflächenaktive Eigenschaften aufweisen.
Daher ist ein weiterer Gegenstand der vorliegenden Erfindung die Verwendung der erfindungsgemäßen modifizierten Siloxane als Emulgator. Oben als erfindungsgemäß bevorzugt dargestellte modifizierte Siloxane der vorliegenden Erfindung werden entsprechend ihrer Bevorzugung erfindungsgemäß bevorzugt verwendet.
Die erfindungsgemäße Verwendung findet bevorzugt in pharmazeutischen oder kosmetischen Formulierungen statt. Dies ist insbesondere daher von Vorteil, da die erfindungsgemäßen modifizierten Siloxane sich frei von reizenden, organischen Lösungsmitteln darstellen lassen und daher am Ende der Herstellung eine hohe Reinheit aufweisen.
Bei der erfindungsgemäßen Verwendung als Emulgator ist es insbesondere bevorzugt, dass das erfindungsgemäße modifizierte Siloxan der allgemeinen Formel I) die Maßgabe erfüllt, dass
- N: = 2 + d + e + f + g+ 2h + 3i = 51 bis 350, bevorzugt 60 bis 250, insbesondere 65 bis 180,
- y: =(a + c + e + g)/ (b + f) = 0,5 bis 25, bevorzugt 2 bis 20, insbesondere3 bis 10, und
- z: = a + b +c + e + f + g = größer/gleich 10, bevorzugt größer 12, insbesondere größer 14,
ist.

Das vorgenannt erfindungsgemäß insbesondere bevorzugt verwendete modifizierte Siloxan ist ein bevorzugtes erfindungsgemäßes Siloxan der vorliegenden Erfindung.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Beispiel 1: Ketalisierung von Glycerinmonoallylether

In einem 1-I- Mehrhalskolben mit KPG-Rührer, Innenthermometer und aufgesetztem Rückflusskühler werden 300g Glycerinmonoallylether GAE (MG: 132,16 g/mol) zusammen mit 354,6g 2,2-Dimethoxypropan DMP (MG: 104,15g/mol; Sdp.: 83°C; 50 % Überschuss) bei 20°C vorgelegt und dann mit 0,33g p-Toluolsulfonsäure p-TSA (0,05% bez. auf GAE und DMP) versetzt und für eine Stunde auf Rückflusstemperatur erhitzt. Der Rückflusskühler wird dann durch eine Destillationsbrücke ersetzt. Es erfolgt im Laufe von 4 Stunden eine langsame Destillatabnahme, wobei die Destillattemperatur bis auf 80°C ansteigt. Nach Erkalten des Sumpfes werden im Laufe von 30 Minuten zur Neutralisation der Säure 13 g Natriumhydrogencarbonat hinzugefügt. Das Salz wird über einen Faltenfilter (MN 615 1/4) abgetrennt und das Filtrat wird am Rotationsverdampfer bei 60°C und einem Druck < 1mbar von Flüchtigen befreit. Das ¹H-NMR-Spektrum der farblos klaren Flüssigkeit belegt quantitative Ketalisierung.

### Beispiel 2: Hydrosilylierung eines Wasserstoffsiloxans mit 1-Dodecen und Glycerinmonoallyletherketal

In einem 1-I- Mehrhalskolben mit KPG-Rührer, Innenthermometer und aufgesetztem Rückflusskühler werden 96,6 g des in Beispiel 1 hergestellten Glycerinmonoallyletherketals 28Val-%; MG: 172,2 g/mol; 30% Überschuss) gemeinsam mit 243,9 g Dodecen (72Val-%; MG: 168,9 g/mol; 30% Überschuss)
bei 20°C unter Rühren vorgelegt und auf 60°C erwärmt. Die Mischung beider Komponenten bildet eine klare Phase. 237,2 mg eines mit Ethylen aktivierten Karstedt-Katalysators gelöst in Dekamethylcyclopentasiloxan gemäß der Lehre der EP 1520870 B1 (1% Pt gelöst in D₅, ethylenbegast entsprechend 3 ppm Pt bezogen auf den Gesamtansatz) werden hinzugegeben und dann werden 450g eines Wasserstoffsiloxans, das sowohl kettenständige als auch terminale SiH-Funktionen besitzt und eine mittlere Kettenlänge N = 120 (SiH-Wert: 3,43 mmol SiH/g) aufweist innerhalb von 30 Minuten hinzugetropft. Die einsetzende Exothermie erwärmt den über die gesamte Zeit der Zudosierung hinweg klar bleibenden Reaktionsansatz auf 90°C. Man lässt 4 Stunden bei 90°C nachreagieren. Die gasvolumetrische SiH-Bestimmung (Zersetzung einer aliquot abgewogenen Probe an einer Gasbürette durch Zugabe von Natriumbutylatlösung und Messung des freigesetzten Wasserstoffvolumens) belegt einen SiH-Umsatz von 99,1%. Das Rohprodukt wird am Rotationsverdampfer bei 130°C und bei einem angelegten Hilfsvakuum von < 1 mbar von Flüchtigen befreit. Es wird eine klare, leicht gelbliche Flüssigkeit isoliert. Ein ¹H-NMR-Spektrum sichert die angestrebte Struktur ab.

### Beispiel 3: Deketalisierung/ Partielle Hydrolyse der in Beispiel 2 hergestellten Vorstufe

In einem 250-ml-Mehrhalskolben mit Innenthermometer und aufgesetztem Rückflusskühler werden 100 g der in Beispiel 2 hergestellten Vorstufe unter Rühren mit 5 g einer 5%igen, wässrigen Toluolsulfonsäurelösung versetzt und unter weiterem, ständigen Rühren für 6 Stunden auf 80°C erwärmt. Nach Abkühlen des Reaktionsgemisches auf 20°C werden zur Neutralisation der Säure 5 g Natriumhydrogencarbonat hinzugefügt. Man lässt diese Mischung ca. 30 Minuten reagieren bevor man mit Hilfe einer Filterpresse (Filterplatte Seitz K300) das Salz abtrennt und das Filtrat bei 80°C und einem angelegten Hilfsvakuum von < 1 mbar von flüchtigen Bestandteilen befreit. Das ¹H-NMR-Spektrum weist dem farblos klaren Produkt einen Deketalisierungsgrad von ca. 83% zu.

In Analogie zu der in den Beispielen 1 bis 3 vorgezeichneten Reaktionsabfolge umfassend die Ketalisierung, die Hydrosilylierung und die Deketalisierung werden die Beispiele 4 bis 8 ausgeführt, wobei jedes Beispiel in der beigefügten Tabelle stellvertretend für die gesamte Reaktionsabfolge steht.
Die Abkürzung **Dk** kennzeichnet den für den individuellen Strukturtyp eingestellten Deketalisierungsgrad, das heißt, das Verhältnis von geöffneter zu ungeöffneter Ketalstruktur in Prozent, wie es sich aus dem jeweils zugehörigen ¹H-NMR-Spektrum bestimmen lässt.

| **Beispiel** | **3** | **4** | **5** | **6** | **7** | **8** |
|---|---|---|---|---|---|---|
| **M_{2+h+2i-a-b-c}** | 2+h+2i-a-b-c = 1 | 2+h+2i-a-b-c = 1 | 2+h+2i-a-b-c = 1 | 2+h+2i-a-b-c = 2 | 2+h+2i-a-b-c = 2 | 2+h+2i-a-b-c = 3 |
| **M'a** | a = 0,725 | a = 0,725 | a = 0,725 | a = 0 | a = 0 | a = 0 |
| **M"_{b}** | b = 0,228 | b = 0,058 | b = 0,052 | b = 0 | b = 0 | b = 0 |
| **M'"_{c}** | c = 0,047 | c = 0,217 | c = 0,222 | c = 0 | c = 0 | c = 0 |
| **D_{d}** | d = 89 | d = 89 | d = 89 | d = 34 | d = 34 | d = 89 |
| **D'ₑ** | e = 21 | e = 21 | e = 21 | e = 10 | e = 10 | e = 21 |
| **D"_{f}** | f = 6,64 | f = 1,68 | f = 1,52 | f = 3,2 | f = 1,68 | f=6,96 |
| **D"'_{g}** | g = 1,36 | g = 6,32 | g = 6,48 | g = 0,8 | g = 2,32 | g = 1,04 |
| **Tₕ** | h = 0 | h = 0 | h = 0 | h = 0 | h = 0 | h=1 |
| **Qᵢ** | i = 0 | i = 0 | i = 0 | i = 0 | i = 0 | i = 0 |
| **R¹** | = CH₃ | = CH₃ | = CH₃ | = CH₃(99%) = TTMSS(1%)³⁾ | = CH₃ | = CH₃ |
| **R²** | = C₁₂H₂₅ | = C₁₂H₂₅ | = C₁₂H₂₅ | = C₁₆H₃₃ | = C₁₂H₂₅ | = C₁₂H₂₅ |
| **R³** | X = CH₂ | X = CH₂ [R'R"]= -(CH₂)₄- ¹⁾ | X = CH₂ [R'R"]= -(CH₂)₅- ²⁾ | X = CH₂ | X = CH₂ | X = CH₂ |
| | R' = CH₃ | | | R' = CH₃ | R' = C₁₅H₃₁ | R' = CH₃ |
| | R" = CH₃ | | | R" = CH₃ | R"= C₁₅H₃₁ | R" = CH₃ |
| **Dk** | 83% | 21% | 19% | 80% | 42% | 87% |

| | | | | | | |
|---|---|---|---|---|---|---|
| ¹⁾ zur Ketalisierung wurde in Beispiel 4 Cyclopentanon eingesetzt ²⁾ zur Ketalisierung wurde in Beispiel 5 Cyclohexanon eingesetzt ³⁾ TTMSS steht für den Tris(trimethylsiloxy)silanethyl-Rest, dessen Präsenz im dendritischen Siloxangerüst als Prozentanteil aller R¹-Reste im Beispiel 6 angegeben ist. | | | | | | |

## Patentansprüche

1. Verfahren zur Herstellung von modifizierten Siloxanen umfassend die Verfahrensschritte
A) Bereitstellen eines Wasserstoffsiloxans aufweisend pro Molekül mindestens drei SiH-Gruppen;
B) Hydrosilylierung des Wasserstoffsiloxans mit
mindestens einem α-olefinischen Kohlenwasserstoff aufweisend 2 bis 36, insbesondere 8 bis 28, Kohlenstoffatome und
mindestens einem ketalisierten Glycerinderivat, welches umfasst:
ein Glyceringerüst und
eine α-olefinischen Kopplungsgruppe; und gegebenenfalls
C) Aufreinigung des hydrosilylierten Siloxans; und gegebenenfalls
D) mindestens teilweise saure Hydrolyse der in den mit dem Wasserstoffsiloxan reagierten ketalisierten Glycerinderivaten enthaltenen Ketale.

2. Verfahren gemäß Anspruch 1 zur Herstellung von modifizierten Siloxanen der allgemeinen Formel I)
M_{2+h+2i-a-b-c} M'a M"_{b} M'"c D_{d} D'e D"_{f} D'"_{g} Tₕ Qᵢ allgemeine Formel I)
mit
M = (R¹₃ Si O_{1/2}),
M' = (R¹₂R² Si O_{1/2}),
M" = (R¹2R³ Si O_{1/2}),
M'" = (R¹₂R⁴ Si O_{1/2}),
D = (R¹₂ Si O_{2/2}),
D' = (R¹R² Si O_{2/2}),
D" = (R¹R³ Si O_{2/2}),
D'" = (R¹R⁴ Si O_{2/2}),
T = (R¹ Si O_{3/2}) und
Q = (Si O_{4/2}),
wobei
R¹ = unabhängig voneinander gleiche oder verschiedene lineare oder verzweigte, gegebenenfalls aromatische Kohlenwasserstoffreste mit 1 bis 16, bevorzugt 1 bis 7, Kohlenstoffatomen, die gegebenenfalls OH- oder Esterfunktionen tragen, oder Tris(trimethylsiloxy)silanethyl, bevorzugt Methyl, Phenyl oder Tris(trimethylsiloxy)silanethyl, insbesondere Methyl oder Tris(trimethylsiloxy)silanethyl,
R² = unabhängig voneinander gleiche oder verschiedene lineare oder verzweigte, gegebenenfalls aromatische Kohlenwasserstoffreste mit 2 bis 36, bevorzugt 8 bis 28, Kohlenstoffatomen, bevorzugt lineare Alkylreste mit 8 bis 28 Kohlenstoffatomen, insbesondere n-Dodecyl oder n-Hexadecyl, und
R³ = unabhängig voneinander gleich oder verschieden, ausgewählt aus glycerinhaltiger Rest,
R⁴ = unabhängig voneinander gleich oder verschieden, ausgewählt aus ketalisierter glycerinhaltiger Rest,
ist,
mit
a = 0 bis 7 , insbesondere 0 bis 3,
b = 0 bis 7, insbesondere 0 bis 3,
c = 0 bis 7, insbesondere 0 bis 3,
d = 10 bis 200, bevorzugt 20 bis 150, insbesondere 50 bis 130,
e = 1 bis 130, bevorzugt 5 bis 80, insbesondere 8 bis 40,
f = 0 bis 75, bevorzugt 1 bis 45, insbesondere 1 bis 25,
g = 0 bis 75, bevorzugt 1 bis 45, insbesondere 4 bis 25,
h = 0 bis 10, bevorzugt 0 bis 5, insbesondere 0
i = 0 bis 5, bevorzugt 0 bis 2, insbesondere 0,
mit der Maßgabe, dass c + g > 0.

3. Verfahren gemäß Anspruch 2 oder 3, **dadurch gekennzeichnet, dass** in Verfahrensschritt B) die Hydrosilylierung des Wasserstoffsiloxans mit einer Mischung enthaltend den α-olefinischen Kohlenwasserstoff und das ketalisierte Glycerinderivat durchgeführt wird.

4. Verfahren gemäß mindestens einem der vorherigen Ansprüche, **dadurch gekennzeichnet, dass** in Verfahrensschritt B) maximal 20 Gew.-%, bevorzugt maximal 10 Gew.-%, insbesondere maximal 5 Gew.-%, Lösungsmittel eingesetzt wird, wobei sich die Gewichtsprozente auf die Summe von Wasserstoffsiloxan, α-olefinischen Kohlenwasserstoff, ketalisiertem Glycerinderivat sowie Lösungsmittel beziehen.

5. Verfahren gemäß mindestens einem der vorherigen Ansprüche, **dadurch gekennzeichnet, dass** das in Verfahrensschritt B) eingesetzte ketalisierte Glycerinderivat sich strukturell durch Ketalisierung mit Substanzen ausgewählt aus der Gruppe
R'-(CO)-R" mit R' und R" gleiche oder verschiedene Kohlenwasserstoffreste, insbesondere Alkylgruppen, mit 1 bis 10 Kohlenstoffatomen, Benzaldehyd, Cyclopentanon, Cyclohexanon und Cycloheptanon,
ableitet.

6. Verfahren gemäß mindestens einem der vorherigen Ansprüche, **dadurch gekennzeichnet, dass** in Verfahrensschritt B) ein ketalisiertes Glycerinderivat eingesetzt wird, welches die allgemeine Formel II) aufweist: 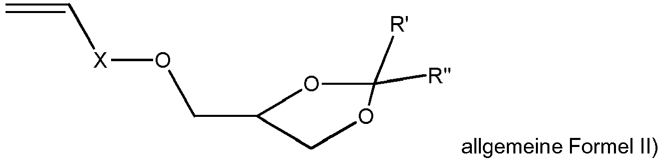
mit R' und R" gleiche oder verschiedene Kohlenwasserstoffreste, insbesondere Alkylgruppen, mit 1 bis 10, insbesondere 1 bis 4, Kohlenstoffatomen, wobei R' und R" kovalent über eine C-C-Bindung miteinander verbunden sein können, und
X ausgewählt aus zweibindigen Kohlenwasserstoffresten, insbesondere Alkylengruppen, mit 1 bis 10 Kohlenstoffatomen, wobei X = CH₂ besonders bevorzugt ist.

7. Verfahren gemäß Anspruch 6, **dadurch gekennzeichnet, dass** in Verfahrensschritt B) ein ketalisiertes Glycerinderivat der allgemeinen Formel II) mit X = CH₂ und R' und R" = CH₃ eingesetzt wird.

8. Verfahren gemäß Anspruch 7 zur Herstellung von Siloxanen der allgemeinen Formel I) in Anspruch 2 mit
R¹ = Methyl,
R² = insbesondere n-Dodecyl oder n-Hexadecyl,
und
a = 0 bis 3,
b = 0 bis 3,
c = 0 bis 3,
d = 50 bis 130,
e = 8 bis 40,
f = 1 bis 25,
g = 0,5 bis 25,
h = 0, und
i = 0.

9. Verfahren gemäß mindestens einem der vorherigen Ansprüche, **dadurch gekennzeichnet, dass** Verfahrensschritt C) derart ausgeführt wird, dass der Ketalisierungsgrad zwischen 5% % und 70%, bevorzugt 10 % und 40 %, besonders bevorzugt 15 % und 25 % liegt, wobei sich die Prozente auf alle im Siloxan vorhandenen, zur Ketalisierung geeigneten Diole beziehen.

10. Verfahren gemäß mindestens einem der vorherigen Ansprüche, weiterhin umfassend Verfahrensschritt D) Aufreinigung des modifizierten Siloxans.

11. Siloxan erhältlich nach einem Verfahren gemäß mindestens einem der vorherigen Ansprüche, **dadurch gekennzeichnet, dass** es mindestens einen ketalisierten glycerinhaltigen Rest aufweist.

12. Siloxan gemäß Anspruch 11, mit allgemeiner Formel I)
M_{2+h+2i-a-b-c} M'a M"_{b} M'"c D_{d} D'e D"_{f} D'"_{g} Tₕ Qᵢ allgemeine Formel I)
mit
M = (R¹₃SiO_{1/2}),
M' = (R¹₂R² Si O_{1/2}),
M" = (R¹₂R³ Si O_{1/2}),
M'" = (R¹₂R⁴ Si O_{1/2}),
D = (R¹₂ Si O_{2/2}),
D' = (R¹R² Si O_{2/2}),
D" = (R¹R³ Si O_{2/2}),
D'" = (R¹R⁴ Si O_{2/2}),
T = (R¹ Si O_{3/2}) und
Q = (Si O_{4/2}),
wobei
R¹ = unabhängig voneinander gleiche oder verschiedene lineare oder verzweigte, gegebenenfalls aromatische Kohlenwasserstoffreste mit 1 bis 16, bevorzugt 1 bis 7, Kohlenstoffatomen, die gegebenenfalls OH- oder Esterfunktionen tragen, oder Tris(trimethylsiloxy)silanethyl, bevorzugt Methyl, Phenyl oder Tris(trimethylsiloxy)silanethyl, insbesondere Methyl oder Tris(trimethylsiloxy)silanethyl,
R² = unabhängig voneinander gleiche oder verschiedene lineare oder verzweigte, gegebenenfalls aromatische Kohlenwasserstoffreste mit 2 bis 36, bevorzugt 8 bis 28, Kohlenstoffatomen, bevorzugt lineare Alkylreste mit 8 bis 28 Kohlenstoffatomen, insbesondere n-Dodecyl oder n-Hexadecyl, und
R³ = unabhängig voneinander gleich oder verschieden, ausgewählt aus glycerinhaltiger Rest,
R⁴ = unabhängig voneinander gleich oder verschieden, ausgewählt aus ketalisierter glycerinhaltiger Rest,
ist,
mit
a = 0 bis 7 , insbesondere 0 bis 3,
b = 0 bis 7, insbesondere 0 bis 3,
c = 0 bis 7, insbesondere 0 bis 3,
d = 10 bis 200, bevorzugt 20 bis 150, insbesondere 50 bis 130,
e = 1 bis 130, bevorzugt 5 bis 80, insbesondere 8 bis 40,
f = 0 bis 75, bevorzugt 1 bis 45, insbesondere 1 bis 25,
g = 0 bis 75, bevorzugt 1 bis 45, insbesondere 4 bis 25,
h = 0 bis 10, bevorzugt 0 bis 5, insbesondere 0
i = 0 bis 5, bevorzugt 0 bis 2, insbesondere 0,
mit der Maßgabe, dass c + g > 0.

13. Siloxan gemäß Anspruch 12 , **dadurch gekennzeichnet, dass**
R¹ = Methyl,
R² = insbesondere n-Dodecyl oder n-Hexadecyl, und
R³ = 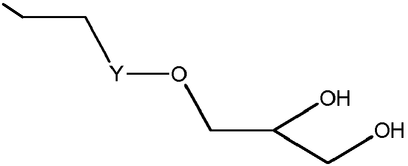 mit Y = [CH₂]ₛ, wobei s = 1 bis 5, insbesondere 1,
R⁴ = 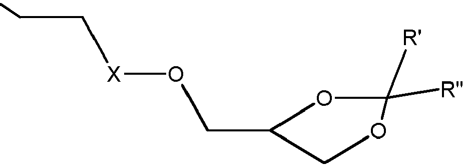 mit X = [CH₂]ᵣ, wobei r = 1 bis 5, insbesondere 1, und R' und R" = unabhängig voneinander gleich oder verschieden, linear Alkyl mit 1 bis 4 Kohlenstoffatomen, wobei R' und R" kovalent über eine C-C-Bindung miteinander verbunden sein können, insbesondere CH₃,
und
a = 0 bis 3,
b = 0 bis 3,
c = 0 bis 3,
d = 50 bis 130,
e = 8 bis 40,
f = 1 bis 25,
g = 0,5 bis 25,
h = 0, und
i = 0.
ist.

14. Verwendung mindestens eines Siloxans gemäß mindestens einem der Ansprüche 11 bis 13 als Emulgator.

15. Verwendung gemäß Anspruch 14 **dadurch gekennzeichnet, dass** das Siloxan der allgemeinen Formel I) die Maßgabe erfüllt, dass
N = 2 + d + e + f + g+ 2h + 3i = 51 bis 350, bevorzugt 60 bis 250, insbesondere 65 bis 180,
y = (a + c + e + g)/ (b + f) = 0,5 bis 25 , bevorzugt 2 bis20 , insbesondere 3 bis 10, und
z = a + b +c + e + f + g = größer/gleich 10, bevorzugt größer 12, insbesondere größer 14,
ist.

16. Verwendung gemäß Anspruch 14 oder 15 in einer pharmazeutischen oder kosmetischen Formulierung.

## Claims

1. Method for preparing modified siloxanes comprising the method steps of
A) providing a hydrogen siloxane having at least three SiH groups per molecule;
B) hydrosilylation of the hydrogen siloxane with
at least one α-olefinic hydrocarbon having 2 to 36, particularly 8 to 28, carbon atoms and
at least one ketalized glycerol derivative comprising:
a glycerol skeleton and
one α-olefinic coupling group; and optionally
C) purifying the hydrosilylated siloxane; and optionally
D) at least partial acidic hydrolysis of the ketals present in the ketalized glycerol derivatives reacted with the hydrogen siloxane.

2. Method according to Claim 1 for preparing modified siloxanes of the general formula I)
M_{2+h+2i-a-b-c} M'ₐ M"_{b} M'"_{c} D_{d} D'ₑ D''_{f} D'"_{g} Tₕ Qᵢ general formula I)
where
M = (R¹₃ Si O_{1/2}),
M' = (R¹₂R² Si O_{1/2}),
M" = (R¹2R³ Si O_{1/2}),
M'" = (R¹₂R⁴ Si O_{1/2}),
D = (R¹₂ Si O_{2/2}),
D' = (R¹R² Si O_{2/2}),
D" = (R¹R³ Si O_{2/2}),
D'" = (R¹R⁴ Si O_{2/2}),
T = (R¹ Si O_{3/2}) and
Q = (Si O_{4/2}),
where
R¹ = mutually independent, identical or different, linear or branched, optionally aromatic hydrocarbon radicals having 1 to 16, preferably 1 to 7, carbon atoms, which optionally bear OH or ester functions, or tris(trimethylsiloxy)silane ethyl, preferably methyl, phenyl or tris(trimethylsiloxy)silane ethyl, in particular methyl or tris(trimethylsiloxy)silane ethyl,
R² = mutually independent, identical or different, linear or branched, optionally aromatic hydrocarbon radicals having 2 to 36 carbon atoms, preferably 8 to 28 carbon atoms, preferably linear alkyl radicals having 8 to 28 carbon atoms, particularly n-dodecyl or n-hexadecyl, and
R³ = mutually independent, identical or different, selected from glycerol-containing radicals,
R⁴ = mutually independent, identical or different, selected from ketalized glycerol-containing radicals,
where
a = 0 to 7, particularly 0 to 3,
b = 0 to 7, particularly 0 to 3,
c = 0 to 7, particularly 0 to 3,
d = 10 to 200, preferably 20 to 150, particularly 50 to 130,
e = 1 to 130, preferably 5 to 80, particularly 8 to 40,
f = 0 to 75, preferably 1 to 45, particularly 1 to 25,
g = 0 to 75, preferably 1 to 45, more particularly 4 to 25,
h = 0 to 10, preferably 0 to 5, especially 0
i = 0 to 5, preferably 0 to 2, particularly 0,
with the proviso that c + g > 0.

3. Method according to Claim 2 or 3, **characterized in that** in method step B) the hydrosilylation of the hydrogen siloxane is carried out with a mixture comprising the α-olefinic hydrocarbon and the ketalized glycerol derivative.

4. Method according to at least one of the preceding claims, **characterized in that** in method step B) at most 20% by weight, preferably at most 10% by weight, in particular at most 5% by weight solvent is used, wherein the percent by weight refers to the sum total of hydrogen siloxane, α-olefinic hydrocarbon, ketalized glycerol derivative and solvent.

5. Method according to at least one of the preceding claims, **characterized in that** the ketalized glycerol derivative used in method step B) is derived structurally by ketalizing with substances selected from the group
R'-(CO)-R" where R' and R" are identical or different hydrocarbon radicals, in particular alkyl groups having 1 to 10 carbon atoms, benzaldehyde, cyclopentanone, cyclohexanone and cycloheptanone.
ableitet.

6. Method according to at least one of the preceding claims, **characterized in that** a ketalized glycerol derivative is used in method step B) having the general formula II): 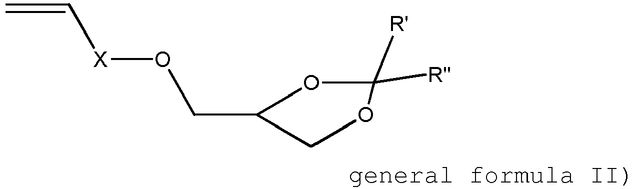
where R' and R" are identical or different hydrocarbon radicals, in particular alkyl groups having 1 to 10, in particular 1 to 4, carbon atoms, wherein R' and R" can be covalently bonded to each other via a C-C bond, and
X is selected from divalent hydrocarbon radicals, in particular alkylene groups having 1 to 10 carbon atoms, where X = CH₂ is particularly preferred.

7. Method according to Claim 6, **characterized in that** in method step B) a ketalized glycerol derivative of the general formula II) is used where X = CH₂ and R' and R" = CH₃.

8. Method according to Claim 7 for preparing siloxanes of the general formula I) in Claim 2 where
R¹ = methyl,
R² = in particular n-dodecyl or n-hexadecyl,
and
a = 0 to 3,
b = 0 to 3,
c = 0 to 3,
d = 50 to 130,
e = 8 to 40,
f = 1 to 25,
g = 0.5 to 25,
h = 0, and
i = 0.

9. Method according to at least one of the preceding claims, **characterized in that** method step C) is carried out such that the degree of ketalization is from 5% to 70%, preferably 10% to 40%, particularly preferably 15% to 25%, wherein the percentage refers to all diols suitable for ketalization present in the siloxane.

10. Method according to at least one of the preceding claims, further comprising method step D) of purifying the modified siloxane.

11. Siloxane obtainable by a method according to at least one of the preceding claims, **characterized in that** said siloxane has at least one ketalized glycerol-containing radical.

12. Siloxane according to Claim 11 with the general formula I)
M_{2+h+2i-a-b-c} M'ₐ M"_{b} M'"_{c} D_{d} D'ₑ D"_{f} D'"_{g} Tₕ Qᵢ general formula I)
where
M = (R¹₃ Si O_{1/2}),
M' = (R¹₂R² Si O_{1/2}),
M" = (R¹2R³ Si O_{1/2}),
M'" = (R¹₂R⁴ Si O_{1/2}),
D = (R¹₂ Si O_{2/2}),
D' = (R¹R² Si O_{2/2}),
D" = (R¹R³ Si O_{2/2}),
D'" = (R¹R⁴ Si O_{2/2}),
T = (R¹ Si O_{3/2}) and
Q = (Si O_{4/2}),
where
R¹ = mutually independent, identical or different, linear or branched, optionally aromatic hydrocarbon radicals having 1 to 16, preferably 1 to 7, carbon atoms, which optionally bear OH or ester functions, or tris(trimethylsiloxy)silane ethyl, preferably methyl, phenyl or tris(trimethylsiloxy)silane ethyl, in particular methyl or tris(trimethylsiloxy)silane ethyl,
R² = mutually independent, identical or different, linear or branched, optionally aromatic hydrocarbon radicals having 2 to 36 carbon atoms, preferably 8 to 28 carbon atoms, preferably linear alkyl radicals having 8 to 28 carbon atoms, particularly n-dodecyl or n-hexadecyl, and
R³ = mutually independent, identical or different, selected from glycerol-containing radicals,
R⁴ = mutually independent, identical or different, selected from ketalized glycerol-containing radicals,
where
a = 0 to 7, particularly 0 to 3,
b = 0 to 7, particularly 0 to 3,
c = 0 to 7, particularly 0 to 3,
d = 10 to 200, preferably 20 to 150, particularly 50 to 130,
e = 1 to 130, preferably 5 to 80, particularly 8 to 40,
f = 0 to 75, preferably 1 to 45, particularly 1 to 25,
g = 0 to 75, preferably 1 to 45, more particularly 4 to 25,
h = 0 to 10, preferably 0 to 5, especially 0
i = 0 to 5, preferably 0 to 2, particularly 0,
with the proviso that c + g > 0.

13. Siloxane according to Claim 12, **characterized in that**
R¹ = methyl,
R² = in particular n-dodecyl or n-hexadecyl, and
R³ = 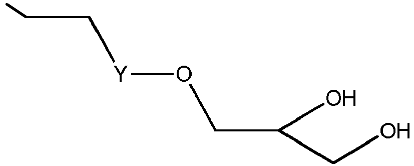 where Y = [CH₂]ₛ, where s = 1 to 5, particularly 1,
R⁴ = 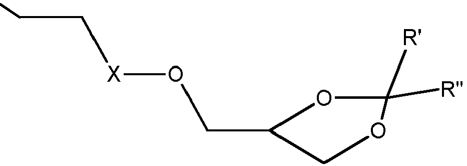 where X = [CH₂]ᵣ, where r = 1 to 5, particularly 1, and R' and R" = mutually independent, identical or different linear alkyl having 1 to 4 carbon atoms, wherein R' and R" can be covalently bonded to each other via a C-C bond, in particular CH₃,
and
a = 0 to 3,
b = 0 to 3,
c = 0 to 3,
d = 50 to 130,
e = 8 to 40,
f = 1 to 25,
g = 0.5 to 25,
h = 0, and
i = 0.

14. Use of at least one siloxane according to at least one of Claims 11 to 13 as emulsifier.

15. Use according to Claim 14, **characterized in that** the siloxane of the general formula I) meets the proviso that
N = 2 + d + e + f + g+ 2h + 3i = 51 to 350, preferably 60 to 250, in particular 65 to 180,
y = (a + c + e + g)/ (b + f) = 0.5 to 25, preferably 2 to 20, in particular 3 to 10, and
z = a + b +c + e + f + g = greater than/equal to 10, preferably greater than 12, in particular, greater than 14.

16. Use according to Claim 14 or 15 in a pharmaceutical or cosmetic formulation.

## Revendications

1. Procédé de fabrication de siloxanes modifiés comprenant les étapes de procédé suivantes :
A) la préparation d'un hydrogénosiloxane comprenant par molécule au moins trois groupes SiH ;
B) l'hydrosilylation de l'hydrogénosiloxane avec
au moins un hydrocarbure α-oléfinique comprenant 2 à 36, notamment 8 à 28, atomes de carbone, et
au moins un dérivé de glycérine cétalisé, qui comprend :
un squelette de glycérine et
un groupe de couplage α-oléfinique ; et éventuellement
C) la purification du siloxane hydrosilylé ; et éventuellement
D) l'hydrolyse acide au moins partielle des cétals contenus dans les dérivés de glycérine cétalisés ayant réagi avec l'hydrogénosiloxane.

2. Procédé selon la revendication 1, pour la fabrication de siloxanes modifiés de formule générale I)
M_{2+h+2i-a-b-c}M'ₐM'_{"b}M'"_{c}D_{d}D'ₑD"_{f}D'"_{g}TₕQᵢ Formule générale I)
avec
M = (R¹₃SiO_{1/2}),
M' = (R¹₂R²SiO_{1/2}),
M" = (R¹₂R³SiO_{1/2}),
M'" = (R¹₂R⁴SiO_{1/2}),
D = (R¹₂SiO_{2/2}),
D' = (R¹R²SiO_{2/2}),
D" = (R¹R³SiO_{2/2}),
D'" = (R¹R⁴SiO_{2/2}),
T = (R¹SiO_{3/2}) et
Q = (SiO_{4/2}),
avec
R¹ = indépendamment les uns des autres, radicaux hydrocarbonés linéaires ou ramifiés, éventuellement aromatiques, identiques ou différents, de 1 à 16, de préférence 1 à 7, atomes de carbone, qui portent éventuellement des fonctions OH ou ester, ou tris(triméthylsiloxy)silanéthyle, de préférence méthyle, phényle ou tris(triméthylsiloxy)silanéthyle, notamment méthyle ou tris(triméthylsiloxy)silanéthyle,
R² = indépendamment les uns des autres, radicaux hydrocarbonés linéaires ou ramifiés, éventuellement aromatiques, identiques ou différents, de 2 à 36, de préférence 8 à 28, atomes de carbone, de préférence radicaux alkyle linéaires de 8 à 28 atomes de carbone, notamment n-dodécyle ou n-hexadécyle, et
R³ = indépendamment les uns des autres, de manière identique ou différente, choisis parmi un radical contenant de la glycérine,
R⁴ = indépendamment les uns des autres, de manière identique ou différente, choisis parmi un radical contenant de la glycérine cétalisé,
avec
a = 0 à 7, notamment 0 à 3,
b = 0 à 7, notamment 0 à 3,
c = 0 à 7, notamment 0 à 3,
d = 10 à 200, de préférence 20 à 150, notamment 50 à 130,
e = 1 à 130, de préférence 5 à 80, notamment 8 à 40,
f = 0 à 75, de préférence 1 à 45, notamment 1 à 25,
g = 0 à 75, de préférence 1 à 45, notamment 4 à 25,
h = 0 à 10, de préférence 0 à 5, notamment 0,
i = 0 à 5, de préférence 0 à 2, notamment 0, à condition que c + g > 0.

3. Procédé selon la revendication 2 ou 3, **caractérisé en ce qu'**à l'étape de procédé B), l'hydrosilylation de l'hydrogénosiloxane est réalisée avec un mélange contenant l'hydrocarbure α-oléfinique et le dérivé de glycérine cétalisé.

4. Procédé selon au moins l'une quelconque des revendications précédentes, **caractérisé en ce qu'**à l'étape de procédé B), au plus 20 % en poids, de préférence au plus 10 % en poids, notamment au plus 5 % en poids, de solvant est utilisé, les pourcentages en poids se rapportant à la somme de l'hydrogénosiloxane, de l'hydrocarbure α-oléfinique, du dérivé de glycérine cétalisé et du solvant.

5. Procédé selon au moins l'une quelconque des revendications précédentes, **caractérisé en ce que** le dérivé de glycérine cétalisé utilisé à l'étape de procédé B) est dérivé structuralement par cétalisation avec des substances choisies dans le groupe :
R'-(CO)-R" avec R' et R" des radicaux hydrocarbonés identiques ou différents, notamment des groupes alkyle, de 1 à 10 atomes de carbone, benzaldéhyde, cyclopentanone, cyclohexanone et cycloheptanone.

6. Procédé selon au moins l'une quelconque des revendications précédentes, **caractérisé en ce qu'**à l'étape de procédé B), un dérivé de glycérine cétalisé est utilisé, qui présente la formule générale II) : 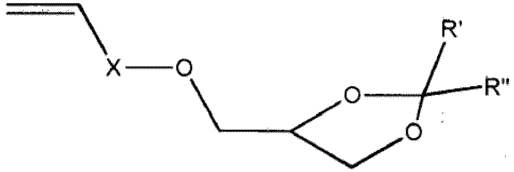 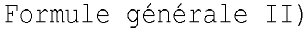
avec R' et R" des radicaux hydrocarbonés identiques ou différents, notamment des groupes alkyle, de 1 à 10, notamment 1 à 4, atomes de carbone, R' et R" pouvant être reliés l'un avec l'autre de manière covalente par une liaison C-C, et
X choisi parmi les radicaux hydrocarbonés bivalents, notamment les groupes alkylène, de 1 à 10 atomes de carbone, X = CH₂ étant particulièrement préféré.

7. Procédé selon la revendication 6, **caractérisé en ce qu'**à l'étape de procédé B), un dérivé de glycérine cétalisé de formule générale II) avec X = CH₂ et R' et R" = CH₃ est utilisé.

8. Procédé selon la revendication 7 pour la fabrication de siloxanes de formule générale I) dans la revendication 2 avec
R¹ = méthyle,
R² = notamment n-dodécyle ou n-hexadécyle,
et
a = 0 à 3,
b = 0 à 3,
c = 0 à 3,
d = 50 à 130,
e = 8 à 40,
f = 1 à 25,
g = 0,5 à 25,
h = 0 et
i = 0.

9. Procédé selon au moins l'une quelconque des revendications précédentes, **caractérisé en ce que** l'étape de procédé C) est réalisée de telle sorte que le degré de cétalisation se situe entre 5 % et 70 %, de préférence entre 10 % et 40 %, de manière particulièrement préférée entre 15 % et 25 %, les pourcentages se rapportant à tous les diols appropriés pour la cétalisation présents dans le siloxane.

10. Procédé selon au moins l'une quelconque des revendications précédentes, comprenant en outre l'étape de procédé D), la purification du siloxane modifié.

11. Siloxane pouvant être obtenu par un procédé selon au moins l'une quelconque des revendications précédentes, **caractérisé en ce qu'**il comprend au moins un radical contenant de la glycérine cétalisé.

12. Siloxane selon la revendication 11, ayant la formule générale I) :
M_{2+h+2i-a-b-c}M'ₐM"_{b}M'"_{c}D_{d}D'ₑD"_{f}D'"_{g}TₕQᵢ Formule générale I)
avec
M = (R¹₃SiO_{1/2}),
M' = (R¹₂R²SiO_{1/2}),
M" = (R¹₂R³SiO_{1/2}),
M'" = (R¹₂R⁴SiO_{1/2}),
D = (R¹₂SiO_{2/2}),
D' = (R¹R²SiO_{2/2}),
D" = (R¹R³SiO_{2/2}),
D'" = (R¹R⁴SiO_{2/2}),
T = (R¹SiO_{3/2}) et
Q = (SiO_{4/2}),
avec
R¹ = indépendamment les uns des autres, radicaux hydrocarbonés linéaires ou ramifiés, éventuellement aromatiques, identiques ou différents, de 1 à 16, de préférence 1 à 7, atomes de carbone, qui portent éventuellement des fonctions OH ou ester, ou tris(triméthylsiloxy)silanéthyle, de préférence méthyle, phényle ou tris(triméthylsiloxy)silanéthyle, notamment méthyle ou tris(triméthylsiloxy)silanéthyle,
R² = indépendamment les uns des autres, radicaux hydrocarbonés linéaires ou ramifiés, éventuellement aromatiques, identiques ou différents, de 2 à 36, de préférence 8 à 28, atomes de carbone, de préférence radicaux alkyle linéaires de 8 à 28 atomes de carbone, notamment n-dodécyle ou n-hexadécyle, et
R³ = indépendamment les uns des autres, de manière identique ou différente, choisis parmi un radical contenant de la glycérine,
R⁴ = indépendamment les uns des autres, de manière identique ou différente, choisis parmi un radical contenant de la glycérine cétalisé,
avec
a = 0 à 7, notamment 0 à 3,
b = 0 à 7, notamment 0 à 3,
c = 0 à 7, notamment 0 à 3,
d = 10 à 200, de préférence 20 à 150, notamment 50 à 130,
e = 1 à 130, de préférence 5 à 80, notamment 8 à 40,
f = 0 à 75, de préférence 1 à 45, notamment 1 à 25,
g = 0 à 75, de préférence 1 à 45, notamment 4 à 25,
h = 0 à 10, de préférence 0 à 5, notamment 0,
i = 0 à 5, de préférence 0 à 2, notamment 0, à condition que c + g > 0.

13. Siloxane selon la revendication 12, **caractérisé en ce que**
R¹ = méthyle,
R² = notamment n-dodécyle ou n-hexadécyle et
R³ = 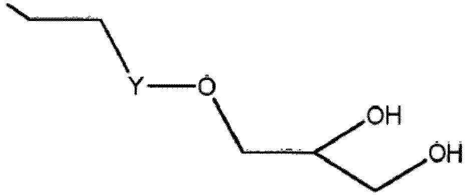 avec Y = [CH₂]ₛ, avec s = 1 à 5, notamment 1,
R⁴ = 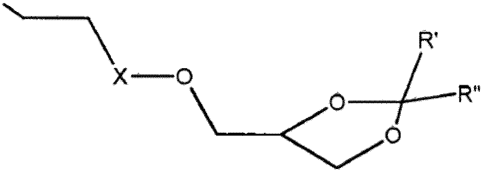 avec X = [CH₂]ᵣ, avec r = 1 à 5, notamment 1, et R' et R" = indépendamment l'un de l'autre, de manière identique ou différente, alkyle linéaire de 1 à 4 atomes de carbone, R' et R" pouvant être reliés l'un avec l'autre de manière covalente par une liaison C-C, notamment CH₃,
et
a = 0 à 3,
b = 0 à 3,
c = 0 à 3,
d = 50 à 130,
e = 8 à 40,
f = 1 à 25,
g = 0,5 à 25,
h = 0 et
i = 0.

14. Utilisation d'au moins un siloxane selon au moins l'une quelconque des revendications 11 à 13 en tant qu'émulsifiant.

15. Utilisation selon la revendication 14, **caractérisée en ce que** le siloxane de formule générale I) remplit les conditions suivantes :
N = 2 + d + e + f + g + 2h + 3i = 51 à 350, de préférence 60 à 250, notamment 65 à 180,
y = (a + c + e + g)/(b + f) = 0,5 à 25, de préférence 2 à 20, notamment 3 à 10, et
z = a + b + c + e + f + g = supérieur ou égal à 10, de préférence supérieur à 12, notamment supérieur à 14.

16. Utilisation selon la revendication 14 ou 15 dans une formulation pharmaceutique ou cosmétique.
